# EUROPEAN PATENT SPECIFICATION

(11) **EP 0 459 377 B1**
(45) Date of publication and mention of the grant of the patent: **11.08.1999**
(21) Application number: 91108655.1
(22) Date of filing: 28.05.1991
(51) Int. Cl.: A61K 31/557

(54) **Use of E1 prostaglandin to cure male erectile inpotence**
Verwendung von Prostaglandin-E1 zur Behandlung männlicher erektiler Impotenz
Utilisation de la prostaglandine E1 pour guérir l'impuissance érectile masculine

(30) Priority: 31.05.1990 IT 8620490
(43) Date of publication of application: 04.12.1991
(73) Proprietor: Reale, Alberto, 20122 Milano (IT)
(72) Inventor: Reale, Alberto, 20122 Milano (IT)
(74) Representative: Gervasi, Gemma, Dr.

(56) References cited:
- DIALOG INFORMATION SERVICES, FILE EMBASE, no. 7551368, embase no. 89273650; P. SCHRAMEK et al.: "Dose-dependent effect and side-effect of prostaglandin E1 in erectile dysfunction", & BR. J. CLIN. PHARMACOL. (UNITED KINGDOM), 1989, 28/5, P567-571
- DIALOG INFORMATION SERVICES, FILE EMBASE, no. 07474908, embase no. 89197170; S. R. ABOSEIF et al.: "Local and systemic effects of chronic intracavernous injection of papaverine, prostaglandin E1, and saline in primates", & J. UROL., 1989.142/4, P. 403-408
- DIALOG INFORMATION SERVICES, FILE EMBASE, no. 8338138, embase no. 92011538; H. PADMA-NATHAN et al.: "Neurogenic erectile dysfunction. Diagnosis and management", & PROBL. UROL. (USA), 1991, 5/4, P527/540
- DIALOG INFORMATION SERVICES, FILE EMBASE, no. 08456391, embase no. 92132059; T. I.-S. HWANG et al.: "Histopathological change of corpora cavernosa after long- term intracavernous injection, & EUR. UROL., 1991, 20/4, P301-306

## Description

The present invention refers to the use of E1 Prostaglandin (Alprostadil) to prepare pharmaceutical compositions to be used in the cure of male erectile impotence, a treatment administered by the introduction of a solution containing a quantity of Alprostadil between 10 and 50 µg in the corpora cavernosa.

Until a few years ago, it was widely held that male impotence, expecially insofar as erectile deficiency was concerned, could be put down to psychogenic causes.

All the best known authors in the field, Kinsey, Masters and Johnson and later Helen Singer Kaplan, were of the opinion that psycho-physiological states were predominantly to blame for male sexual dysfunctions. It is held that depression, stress and fatigue could have a serious effect on sexuality and male erections in particular although the exact mechanism through which emotional states negatively influence sexuality can not be clearly identified.

However, it has been found that many illnesses, even in their initial stages, can depress the libido, and that hepatopathic or renal deseases, as well as multiple sclerosis and diabetes, negatively influence the erectile function of the male member.

Recent studies have established that the incidence of organic causes on male erectile impotence is certainly not less than 80% and that, amongst these causes, the vascular ones are prevalent. In particular, it was possible to establish using modern vascular diagnosis techniques, which do not require samples or surgery (Doppler method), that primitive alterations of the penis area and alterations of the venous drainage are amongst the prevailing causes.

Aside from those cases when a carvernougraphy shows alterations of the deep dorsal vein, which can be cured by a simple ligature under surgery, it should be remembered that in some instances erection deficiency has been created by papaverin infusion in the patient's corpora cavernosa. It is common knowledge, however, that this treatment may be highly dangerous as there are frequent cases where drugs have had to be administered to patients to counteract prolongued erection. Moreover, it has also been found that the continued use of papaverin can have irreparable delayed consequences (fibrosis of the corpora cavernosa), as well as so called immediate reactions such as priapism. Therefore, this practice has been almost completely abandoned.

Our invention proposes the use of E1 Prostaglandin (Alprostadil) to prepare pharmaceutical compositions for the treatment of male erectile impotence, characterized in that said compositions consist of low alcohol content alcohol solutions of Alprostadil suitable to be introduced into the corpora cavernosa by local injection or by iontophoresis. The pharmaceutical preparations are introduced in quantities of between 10 and 50 µg of the active ingredient in the corpora cavernosa. The results are identical to those obtained using papaverin without any of the immediate or delayed harmful side-effects.

Only a slight swelling is reported in the area where the introduction has been made. Alprostadil (Alprostadilum) is a well known substance usually defined as Alfa blocking:
11,15- Dihydroxy-9-oxoproxt-13-en-1-oic acid; 3-hydroxy-2-(3-hydroxy-1-octenyl) -5-oxocyclopentaneheptanoic acid; C₂₀H₃₄O₅ molecular weight 354.49

So far, it has only been used in the following instances:
palliative therapy to keep the arterial duct temporarily open until adequate surgery can be carried out (so-called 'blue-disease') (J.S. Donahoo et al., J. Thoracic Cardiovasc. Surg. 81, 227 (1981); other circulatory disorders like atresia and stenosis of the pulmonary valve, atresia of the tricuspid valve, stenosis and atresia of the aorta valve, and, lastly, in the cure of cronic obstruction of arteries at the third and fourth level.

Our invention proposes a totally new use for Alprostadil and more precisely its use in the preparation of pharmaceutical compositions to cure erectile deficiency. The above mentioned compositions are obtained by dissolving 10 to 50 µg of Alprostadil every 1 to 5 ml of low alcoholic solution.

Each dose contains 10 to 50 µg of Alprostadil.

The therapeutic treatment is carried out by local injection of said compositions in the corpora cavernosa using insulin needles or, better still, micro needles of reduced diameter. Alternatively, said compositions can be introduced using the well known iontophoresis technique.

Observations carried out on a suitable number of patients have shown that, after a period not longer than fifteen minutes, the penis becomes rigid for a period of time between 30 minutes and 4 hours approximately, depending on the quantity of composition introduced and the state of the corpora cavernosa, so as to allow one or more satisfactory acts of sexual intercourse.

Moreover, this method has been found to have no harmful side effects, while having very interesting therapeutic properties, even in the long term, as the corpora cavernosa acquire more elasticity a little at a time, by continually absorbing the composition.

As previously mentioned, Alprostadil is as effective as papaverin in the cure of male erectile deficiencies, while being absolutely free from both immediate and delayed harmful consequences.

Alprostadil, moreover, unlike papaverin, determines more elasticity in the corpora cavernosa in the long term, with obvious advantages.

## Claims

1. Use of Prostaglandin E1 (Alprostadil) in the preparation of pharmaceutical compositions for the treatment of male erectile impotence, characterized in that said compositions consist of low alcohol content alcohol solutions of Alprostadil suitable to be introduced in the corpora cavernosa by local injection or by iontophoresis.

2. Use as per claim 1, characterized in that said compositions consist of 10 to 50 µg of Alprostadil and 1 to 5 ml of low alcohol content alcohol solution.

## Patentansprüche

1. Verwendung von Prostaglandin E1 (Alprostadil) zur Herstellung von pharmazeutischen Zusammensetzungen für die Behandlung der männlichen erektilen Impotenz, dadurch gekennzeichnet, daß die genannten Zusammensetzungen aus alkoholischen Lösungen von Alprostadil mit einem niedrigen Alkohol-Gehalt bestehen, die geeignet sind für die Einführung in die Schwellkörper durch lokale Injektion oder durch Iontophorese.

2. Verwendung nach Anspruch 1, dadurch gekennzeichnet, daß die genannten Zusammensetzungen aus 10 bis 50 µg Alprostadil und 1 bis 5 ml einer alkoholischen Lösung mit einem niedrigen Alkohol-Gehalt bestehen.

## Revendications

1. Utilisation de prostaglandine E1 (Alprostadil) dans la préparation de compositions pharmaceutiques pour le traitement de l'impuissance érectile masculine, caractérisée en ce que lesdites compositions sont constituées par des solutions alcooliques d'Alprostadil, à faible teneur en alcool, appropriées pour être introduites dans le corps caverneux par injection locale ou par iontophorèse.

2. Utilisation selon la revendication 1, caractérisée en ce que lesdites compositions sont constituées par 10 à 50 µg d'Alprostadil et 1 à 5 ml de solution alcoolique à faible teneur en alcool.
